# EUROPEAN PATENT APPLICATION

(11) **EP 4 424 244 A1**
(43) Date of publication of application: **04.09.2024**
(21) Application number: 23159252.8
(22) Date of filing: 28.02.2023
(51) Int. Cl.: A61B 17/00, A61B 90/00

(54) **A SYSTEM FOR DELIVERING A HAEMOSTATIC AGENT**

(71) Applicant: Baxter Medical Systems GmbH + Co. KG, 07318 Saalfeld (DE)
(72) Inventor: SYLVESTER, Jordan, 07318 Saalfeld (DE); EMMONS, Kirsten M., 07318 Saalfeld (DE); TAMBORINI, Janet, 07318 Saalfeld (DE); DALBERT, Heinz-Hermann, 07318 Saalfeld (DE)
(74) Representative: Reddie & Grose LLP

(57) **Abstract**

A system (400) for delivering a haemostatic agent (412) is provided. The system comprising a minimally invasive surgical, MIS, instrument (200) comprising a tube channel (204) and a tube (100) receivable in the tube channel. The tube comprises two fluidically separated flow channels, so that efflux from at least two syringe outlets is prevented from combining before reaching a distal end of the tube. A tube for use in the system, and a kit of parts, are also provided.

## Description

### TECHNICAL FIELD

The present disclosure relates to a system for delivering a haemostatic agent. In particular, the disclosure relates to a system comprising a minimally invasive surgical, MIS, instrument comprising a tube channel and a tube receivable in the tube channel. The tube comprises two fluidically separated flow channels, so that efflux from at least two syringe outlets is prevented from combining before reaching a distal end of the tube.

### BACKGROUND

During surgery, incisions are made to investigate or treat pathological conditions. Minimally invasive surgery has been developed to accelerate and improve recovery from surgery, as well as reduce risk of infection and complications. Minimally invasive surgery includes surgical procedures which are performed minimizing the size and number of incisions.

A variety of minimally invasive surgical, MIS, instruments are known in the literature. When making incisions using a MIS instrument during surgery, it may be desirable to apply a haemostatic agent, or other substances which promote wound closure, promote wound healing, and/or reduce bleeding.

To provide the haemostatic agent to the incision, the MIS instrument must be removed, risking tissue damage and other complications. It has also been found that catheters or (micro)tubes used for delivering haemostatic agents to wounds can become occluded during use, and may thus be suitable only for a single application of a haemostatic agent.

The inventors have appreciated the need for a system which facilitates application of a haemostatic agent during minimally invasive surgery. The inventors have further appreciated the need for a tube for a MIS instrument which may be used to apply a haemostatic agent repeatedly.

### SUMMARY OF THE DISCLOSURE

The present disclosure provides a system for delivering a haemostatic agent, a tube for use in the system, and a kit of part, as defined in the appended claims, to which reference should now be made. Optional features are defined in the enclosed dependent claims. According to a first aspect of the present disclosure, there is provided a system for delivering a haemostatic agent, comprising: a minimally invasive surgical, MIS, instrument comprising a tube channel; and a tube receivable in the tube channel. The tube comprises: a first flow channel; a second flow channel, fluidically separated from the first flow channel; and a connector, at a proximal end of the tube, connectable to at least two syringe outlets.

A first one of the at least two syringe outlets is connectable to the first flow channel, and a second one of the at least two syringe outlets is connectable to the second flow channel, so that efflux from the at least two syringe outlets is prevented from combining before reaching a distal end of the tube.

By preventing efflux from each of two syringe outlets from mixing, or combining, before reaching the distal end of the tube, the haemostatic agent is not formed from the combined effluxes until they reach the distal end of the tube. This may allow for the mixing process to occur only at the (distal) tip of the tube, so that the haemostatic agent can be applied at different times (that is, repeatedly) without occluding the (distal) outlet of the tube.

Additionally, by only allowing mixing at the distal tip of the tube, the formed haemostatic agent may be applied more precisely, i.e., at the desired position and in the desired amount, and without having to remove the MIS instrument to apply the haemostatic agent, owing to the tube channel in the MIS instrument.

As used herein, the term "distal" refers to portions of the system, tube, or MIS instrument positioned towards the outlet/tip, which is intended to contact, or be adjacent to, an incision, or wound, during surgery, while the term "proximal" refers to portions of the system, tube, or MIS instrument that are further from the incision (or wound) during surgery and closer to, for example, the hand of the user or the robotic manipulator holding or engaging the system.

The tube may be made of any suitable type of material. The tube may be flexible. Polymeric materials, such as plastics, in particular sterile plastics, may be suitable materials for the tube.

The tube may be referred to as a catheter, or as a microtube.

Optionally, the tube when received within the tube channel is movable between a first configuration, in which a distal end of the tube is contained within the tube channel, and a second configuration, in which the distal end of the tube projects, in a distal direction, from a distal end/outlet of the tube channel.

In other words, in the second configuration, the distal end of the tube extends beyond, and out of, the distal end of the tube channel.

Advantageously, this may allow the haemostatic agent to be applied more precisely, e.g., exactly where required, when in the second configuration, without impeding the function of the MIS instrument, when in the first configuration. Allowing the tube to be retracted into the tube channel may also reduce any risk of damage to the tube.

Optionally, the system comprises a limiter configured so that the distal end of the tube projects from the distal end of the tube channel by a distance, X.

The limiter may be configured to prevent a distal end of the tube from entering the tube channel, ensuring that the tube may easily be withdrawn by a user.

The distance, X, may be any suitable distance which permits a haemostatic agent to be applied to an incision site when in the second configuration.

Optionally, the limiter is configured so that the distance X is adjustable.

For example, the limiter may comprise a movable element, configured to be moved along the length of the tube and to be secured to the tube. Alternatively, the limiter may comprise a fastener, such as a screw, on the MIS instrument, which may selectively engage the tube to prevent movement of the tube in the tube channel. Further alternatively, the limiter may comprise a tensioning means configured to be selectively tensioned, which in a tensioned state prevents movement of the tube in the tube channel.

The tube may comprise the limiter, and the limiter may be configured to engage with a proximal end of the tube channel upon the tube being in the second configuration. Advantageously, providing such a "passive" limiter on the tube may allow for tubes having differing lengths, or tubes with limiters at different positions along the respective tube, to be provided which, in the second configuration, project different distances X from the distal end of the tube channel. Additionally, a "passive" limiter, which does not have moving parts, may be more reliable than a limiter comprising e.g. tensioning means or fasteners etc.

Optionally, the limiter comprises a flange having a diameter greater than a diameter of the tube channel. Advantageously, such a simple flange may strengthen the tube at the same time as being configured to act as a "passive" limiter.

The limiter may prevent a proximal end portion of the tube from being receivable in the tube channel.

The MIS instrument comprises, at a distal end, a surgical tool. Optionally, the surgical tool comprising a pair of jaws. Advantageously, a surgical tool having a pair of jaws and a retractable tube with two flow channels may synergise to provide a system which can be used for micro surgery, and repeated and selective application of a haemostatic agent.

In the second configuration, the distal end of the tube may project distally from the surgical tool. Advantageously, in this manner, the MIS instrument may more easily be kept in place during application of the haemostatic agent.

The tube may be separable from the MIS instrument. In other words, the MIS instrument and the tube of the system may be separate components, which may be separated from one another.

Advantageously, this may facilitate cleaning of the MIS instrument, whilst the tube may be provided as a disposable component, thus ensuring that the same MIS instrument, with a disposable tube, may easily be used in a sterile manner.

The tube channel may alternatively be referred to as a conduit for the tube. The tube channel preferably extends from a proximal end of the MIS instrument to a distal end of the MIS instrument.

Optionally, the tube channel extends substantially along a longitudinal axis of the MIS instrument. Advantageously, this may facilitate precise application of the haemostatic agent.

Alternatively, the tube channel may extend through the MIS instrument at an angle, relative to the longitudinal axis of the MIS instrument.

The connector may be a standardised connector. The connector may be a Luer lock connector. Advantageously, a Luer lock connector may allow for a syringe to be securely connected to the first and second flow channels.

Optionally, the system further comprises a syringe. The syringe may comprise: a first chamber having a first fluid outlet coupled to the first flow channel, the first chamber containing a first constituent; and a second chamber having a second fluid outlet coupled to the second flow channel, the second chamber containing a second constituent. As such, the syringe may be a dual-chamber, or dual-lumen, syringe, in which the respective constituents in the first and second chambers are prevented from mixing in the syringe and may only combine after they have passed through the respective fluid outlets.

The first constituent and second constituent, when combined, may form a haemostatic agent. The term "haemostatic agent" broadly relates to at least haemostats, sealants and adhesives, that is, to any substance suitable for promoting wound healing or wound closure or which reduces bleeding.

The haemostatic agent may be a fibrin glue. Alternatively, the haemostatic agent may be a gelatine-based sealant. Alternatively, the haemostatic agent may be a glutaraldehyde-based adhesive.

The first constituent may comprise at least one of: fibrinogen, aprotinin, fibronectin, plasminogen, factor XIII, thrombin, calcium, serum albumin, glutaraldehyde; and the second constituent may comprise at least one of: fibrinogen, aprotinin, fibronectin, plasminogen, factor XIII, thrombin, calcium, serum albumin, glutaraldehyde.

A diameter dₜ of the tube may be about 1 mm to about 10 mm, optionally it may be about 2 mm to about 5 mm, and further optionally it may be about 3 mm to about 4 mm.

A diameter d_{tc} of the tube channel may be about 1.5 mm to about 12 mm, optionally it may be about 2.5 to about 6 mm, and further optionally it may be about 3 mm to about 4.5 mm.

The diameter d_{tc} may be substantially the same as the diameter dₜ. This may allow for the tube to be held substantially in place by friction.

In some embodiments, the MIS instrument is locked when the distal end of the tube projects from a, or the, distal end of the tube channel. In other words, the MIS instrument may be prevented from performing surgical functions when the tube is in the second configuration. This may prevent damage to the MIS instrument and/or the tube.

If the limiter is a screw or a tensioning means, the screw or tensioning means may be configured to lock a MIS instrument if the tube is in the second configuration. For example, if the MIS instrument comprises a pair of jaws, the system may be configured so that, when the tube is in the second configuration, the limiter is configured to hold the pair of jaws in an open configuration, preventing them from closing.

According to a second aspect of the present disclosure, there is provided a tube for use in the system according to the first aspect. The tube is receivable in a tube channel of a MIS instrument and comprises: a first flow channel; a second flow channel, fluidically separated from the first flow channel; and a connector, at a proximal end of the tube, connectable to at least two syringe outlets. A first of the at least two syringe outlets is connectable to the first flow channel, and a second of the at least two syringe outlets is connectable to the second flow channel, so that efflux from the at least two outlets is prevented from combining before reaching a distal end of the tube.

By preventing efflux from each of two syringe outlets from mixing, or combining, before reaching the distal end of the tube, the haemostatic agent is not formed from the combined effluxes until it reaches the distal end of the tube. This may allow for the mixing process to occur only at the (distal) tip of the tube, so that the haemostatic agent can be applied at different times without occluding the (distal) outlet of the tube.

Additionally, by only allowing mixing at the distal tip of the tube, haemostatic agent may be applied more precisely, i.e., at the desired position and in the desired amount.

Optionally, the tube comprises a limiter configured to engage with a proximal end of a tube channel of a MIS instrument. Further optionally, the tube is configured to project, distally, from a tube channel of a MIS instrument when the limiter abuts a proximal end of a tube channel of the MIS instrument.

The limiter may comprise a flange having a diameter greater than a diameter of the tube channel.

Optionally, a diameter of the tube is about 1 mm to about 10 mm, optionally about 2 mm to about 5 mm, and further optionally about 3 mm to about 4 mm.

The connector may be a standardised connector. The connector may be a Luer lock connector. Advantageously, a Luer lock connector may allow for a syringe to be securely connected to the first and second flow channels.

It is noted that while preferably, the first syringe outlet and the second syringe outlet are outlets of a dual-chamber syringe, they may also be single outlets of separate single-chamber syringes.

According to a third aspect of the present disclosure, there is provided a kit of parts, comprising a tube according to the second aspect, and a syringe comprising: a first chamber having a first fluid outlet coupled to the first flow channel, the first chamber containing a first constituent; and a second chamber having a second fluid outlet coupled to the second flow channel, the second chamber containing a second constituent.

The first constituent and the second constituent, when combined, may form a haemostatic agent. Alternatively, the first constituent and second constituent when combined may form any other substance suitable for promoting wound healing or wound closure or which reduces bleeding, such as an adhesive.

According to a fourth aspect of the present disclosure, there is provided a method of dispensing a haemostatic agent at a surgical site, comprising: providing a system according to the first aspect; connecting the first flow channel to a first syringe outlet, and providing a first constituent via the first flow channel; connecting the second flow channel to a second syringe outlet, and providing a second constituent via the second flow channel; and mixing the first constituent and the second constituent at a distal tip of the tube to form a haemostatic agent.

Further features of the second, third, and fourth aspects of the present disclosure are described above in relation to the first aspect of the present disclosure.

It will be appreciated that features described in relation to one aspect of the present disclosure may also be applied equally to all of the other aspects of the present disclosure. Features described in relation to the first aspect of the present disclosure may be applied equally to the second, third, or fourth aspect of the present disclosure and vice versa. For example, apparatus features described in relation to the first aspect may be applied, mutatis mutandis, to the tube of the second aspect.

### BRIEF DESCRIPTION OF DRAWINGS

The disclosure will be further described, by way of example only, with reference to the accompanying drawings, in which:
Figure 1 shows a schematic side view of a tube according to the present disclosure;
Figure 2 shows a schematic side view of a minimally invasive surgical, MIS, instrument according to the present disclosure;
Figure 3 shows a schematic side view of a system for delivering a haemostatic agent according to the present disclosure, in a first configuration;
Figure 4A shows a schematic side view of the system of Figure 3 in a second configuration;
Figure 4B shows an enlarged schematic portion of Figure 4A;
Figure 5A shows a cross section of a tube according to the present disclosure; and
Figure 5B shows a cross section of an alternative tube according to the present disclosure.

### DETAILED DESCRIPTION OF DRAWINGS

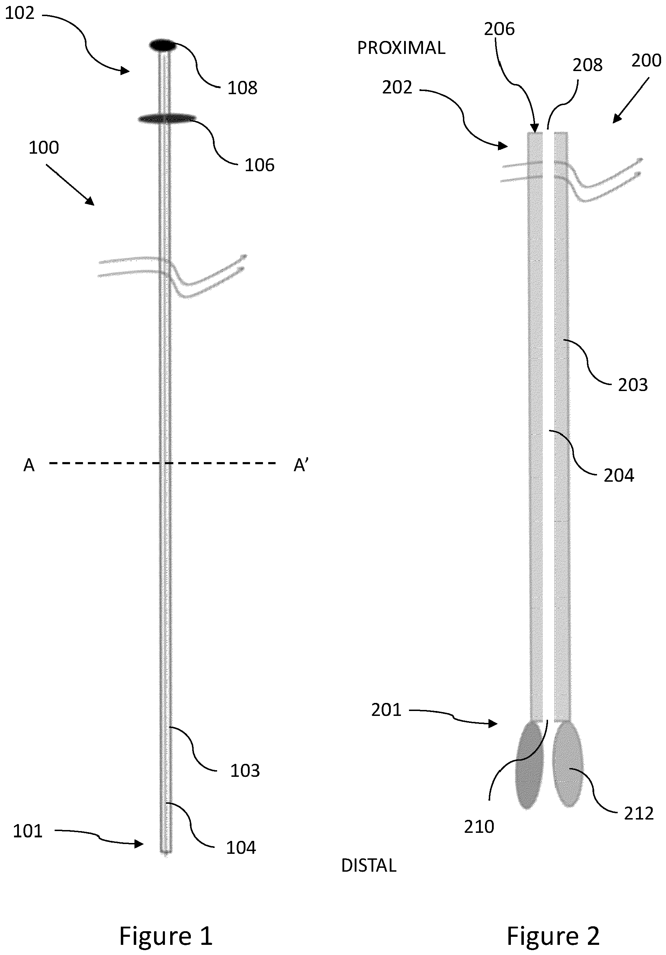

Figure 1 shows a schematic side view of a tube 100, or catheter, for a minimally invasive surgical, MIS, instrument. The tube 100 has a distal end 101 and a proximal end 102. The tube 100 has an outer wall 103. The outer wall surrounds a cavity, or lumen 104, extending from the distal end 101 of the tube 100 to the proximal end 102 of the tube 100.

Towards its proximal end 102, the tube 100 comprises a stopper 106, or limiter, formed of a perpendicular flange surrounding the outer wall 103. The tube 100, at its proximal end 102, comprises a Luer lock connector 108 for fluidly connecting the cavity 104 to an outlet of a syringe.

Figure 2 shows a schematic side view of a MIS instrument 200, having a distal end 201 and a proximal end 202. The MIS instrument 200 comprises a cylindrical portion 203 extending from the distal end 201 towards the proximal end 202. The cylindrical portion 203 comprises a central, cylindrical, tube channel 204 for receiving a tube, such as tube 100, for dispensing a haemostatic agent.

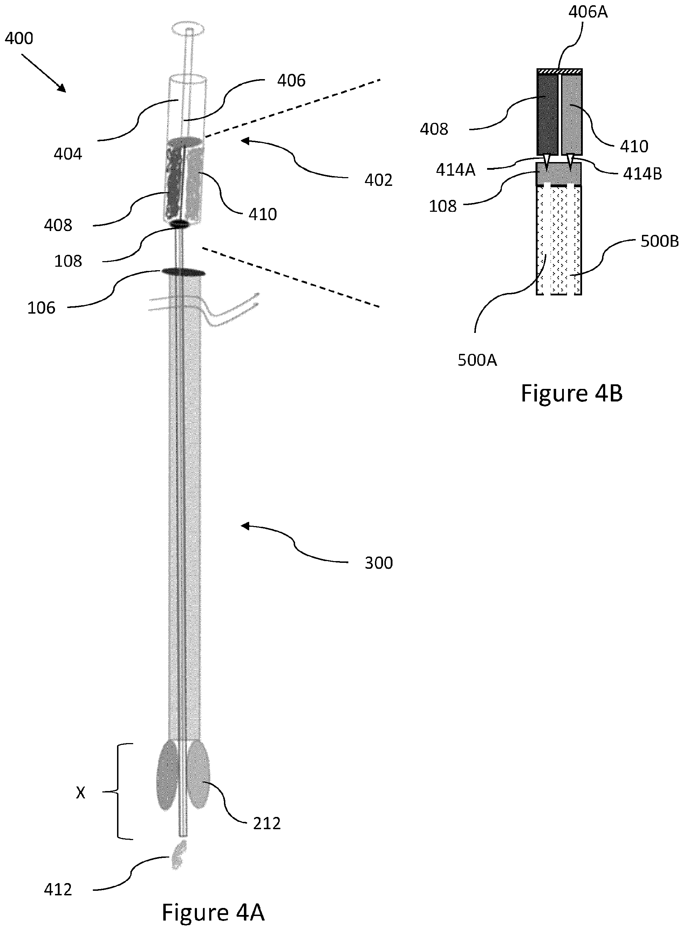

At the proximal end 202, the MIS instrument 200 has an end surface 206, which the flange 106 of the tube 100 is configured to abut. When the tube 100 is being received in the tube channel 204, and the flange 106 abuts the proximal end surface 206 of the MIS instrument 200, the tube 100 extends through an inlet 208 of the tube channel 204 and projects distally from the (distal) outlet 210 of the tube channel 204, as discussed in more detail below with regards to Figure 4A.

At the distal end 201, the MIS instrument 200 comprises a pair of jaws 212, configured for performing minimally invasive surgery.

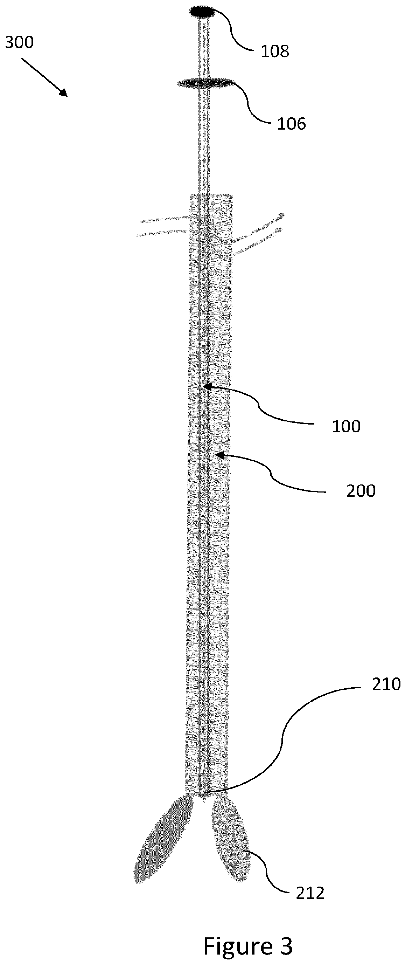

As shown in Figure 3, in a first configuration of a system 300 comprising the tube 100 and the MIS instrument 200, the tube 100 is received within the tube channel 204 of the MIS instrument 200, but does not project distally from the distal outlet 210 of the tube channel 204.

An outer diameter of the tube 100 and an inner diameter of the tube channel 204 is substantially identical. Thus, friction between the outer wall 103 of the tube 100 and the inner wall of the tube channel 204 holds the tube 100 in the first configuration shown in Figure 3.

Figure 4A shows a system 400 comprising the system 300 in a second configuration, in which the tube 100 projects, by a distance X, distally from the distal outlet 210 of the tube channel 204. In the second configuration of the tube 100, the limiter 106, or stopper, abuts the proximal end surface 206 of the MIS instrument 200, which also forms a proximal end of the tube channel 204. This prevents a proximal end 102 of the tube 100 from entering the tube channel 204.

The position of the limiter 106 along the tube 100 relative to a length of the tube and a length of the tube channel 204 of the MIS instrument 200 determines the distance X by which the tube 100 projects distally from the distal outlet 210 of the tube channel 204.

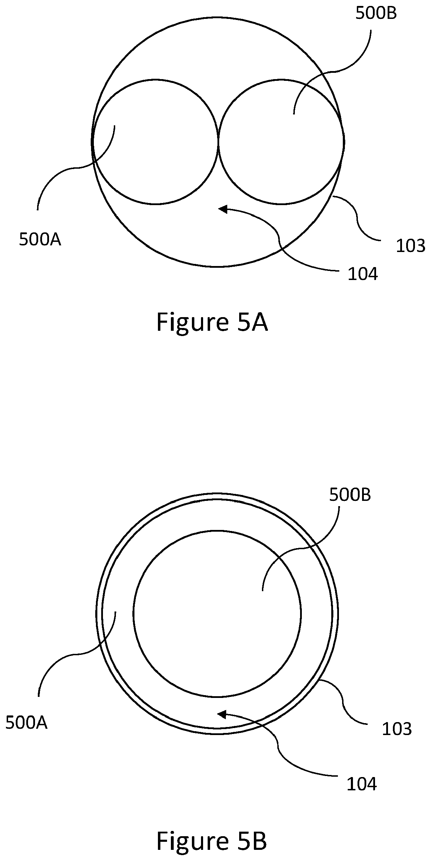

As shown in Figures 5A and 5B, within the cavity 104 of the tube 100, the tube 100 comprises a first flow channel 500A and a second flow channel 500B. The first flow channel 500A and the second flow channel 500B may be arranged in any suitable configuration, such as side by side (see Figure 5A) or co-axially (see Figure 5B), as long as they are fluidically separated from one another.

Referring again to Figure 4A, the system 400 further comprises a syringe 402, connected to the Luer lock connector 108 of the tube 100. The syringe 402 comprises an interior 404, and a plunger 406 movable within the interior 404 to force material from the syringe 402. The interior 404 comprises a first chamber 408 and a second chamber 410. The first and second chambers 408, 410 are fluidically separated from one another, thus preventing the contents within the chambers 408, 410 from mixing.

The first chamber 408 terminates in a first fluid outlet 414A (as shown in the enlarged, schematic box diagram of Figure 4B), which is coupled, via the connector 108, to the first flow channel 500A. The first chamber 408 contains a first constituent of a haemostatic agent. The second chamber 410 terminates at a second fluid outlet 414B (as also shown in the enlarged, schematic box diagram of Figure 4B), which is coupled, via the connector 108, to the second flow channel 500B. The second chamber 410 contains a second constituent of a haemostatic agent.

An end surface 406A of the plunger 406 is movable along the barrel of the syringe 402 to force the constituents of the first chamber 408 and the second chamber 410 out of the respective first and second fluid outlets 414A, 414B.

The first and second fluid outlets 414A, 414B are connected to the tube 100, via the Luer lock connector 108, in such a manner that the first chamber 408 is in fluid communication, via the first fluid outlet 414A, with the first flow channel 500A of the tube 100, and the second chamber 410 is in fluid communication, via the second fluid outlet 414B, with the second flow channel 500B of the tube 100.

The first constituent is fibrinogen. The second constituent is thrombin. The two constituents, when mixed at a tip of the tube 100, form a haemostatic agent 412, i.e. a substance suitable for promoting wound healing or wound closure. In this example, the haemostatic agent 412 is a fibrin glue. At least one of the first constituent and the second constituent may further comprise calcium.

In another example, the first constituent is glutaraldehyde, and the second constituent is serum albumin, in particular purified bovine serum albumin. In this example, when mixed at the tip of the tube 100, the two constituents form a glutaraldehyde-based adhesive.

In use, the syringe 402 is attached to the tube 100 using the Luer lock connector 108. The tube 100, before or after attachment of the syringe 402, is inserted into the tube channel 204 of the MIS instrument 200. The tube 100 may be held in the first configuration, shown in Figure 3, while the surgical tool 212 of the MIS instrument 200 is being used for a surgical procedure.

Upon the haemostatic agent being required at the surgical site, the tube 100 which is held in position in the tube channel 204 by friction between an exterior surface of the outer wall 103 of the tube 100 and an interior surface of the tube channel 204, may be pushed into a distal direction, into the second configuration. In the second configuration, the distal end 101 of the tube 100 projects distally beyond a distal end 201 of the MIS instrument 200, out of the distal outlet 210 of the tube channel 204.

To facilitate precise application of the haemostatic agent 412, the tip of the tube 100 projects beyond the distal end of the pair of jaws 212. Once the haemostatic agent 412 has been applied, the tube 100 may be retracted into the tube channel 204, allowing the surgical tool 212 to be used again.

As the first and second constituents are prevented from mixing before reaching the distal end 101 of the tube 100, the haemostatic agent 412 does not occlude the tube 100, and the process described above may be repeated, allowing haemostatic agent 412 to be repeatedly applied via the tube 100.

It will be understood that the present disclosure has been described above purely by way of example, and modifications of detail can be made within the scope of the invention.

Each feature disclosed in the description, and (where appropriate) the claims and drawings may be provided independently or in any appropriate combination.

## Claims

1. A system for delivering a haemostatic agent, comprising:
a minimally invasive surgical, MIS, instrument comprising a tube channel; and
a tube receivable in the tube channel, the tube comprising:
a first flow channel;
a second flow channel, fluidically separated from the first flow channel; and
a connector, at a proximal end of the tube, connectable to at least two syringe outlets,
a first one of the at least two syringe outlets being connectable to the first flow channel, and a second one of the at least two syringe outlets being connectable to the second flow channel, so that efflux from the at least two syringe outlets is prevented from combining before reaching a distal end of the tube.

2. A system according to claim 1, wherein the tube when received within the tube channel is movable between a first configuration, in which a distal end of the tube is contained within the tube channel, and a second configuration, in which the distal end of the tube projects from a distal end of the tube channel.

3. A system according to claim 2, wherein the system comprises a limiter configured so that the distal end of the tube projects from the distal end of the tube channel by a distance, X.

4. A system according to claim 3, wherein the limiter is configured so that the distance X is adjustable.

5. A system according to claim 3 or 4, wherein the tube comprises the limiter, and the limiter is configured to engage with a proximal end of the tube channel upon the tube being in the second configuration.

6. A system according to claim 5, wherein the limiter comprises a flange having a diameter greater than a diameter of the tube channel.

7. A system according to any preceding claim, wherein the MIS instrument comprises, at a distal end, a surgical tool; and optionally wherein the surgical tool comprises a pair of jaws.

8. A system according to claim 7 when dependent on any of claims 2 to 6, wherein, in the second configuration, the distal end of the tube extends distally beyond the surgical tool.

9. A system according to any preceding claim, wherein the tube is separable from the MIS instrument.

10. A system according to any preceding claim, wherein:
the tube channel extends substantially along a longitudinal axis of the MIS instrument; and/or
the connector is a Luer lock connector.

11. A system according to any preceding claim, further comprising a syringe, the syringe comprising:
a first chamber having a first fluid outlet coupled to the first flow channel, the first chamber containing a first constituent; and
a second chamber having a second fluid outlet coupled to the second flow channel, the second chamber containing a second constituent,
wherein the first constituent and second constituent, when combined, form a haemostatic agent; and optionally wherein the first constituent comprises at least one of: fibrinogen, aprotinin, fibronectin, plasminogen, factor XIII, thrombin, calcium, serum albumin, glutaraldehyde; and wherein the second constituent comprises at least one of: fibrinogen, aprotinin, fibronectin, plasminogen, factor XIII, thrombin, calcium, serum albumin, glutaraldehyde.

12. A tube for use in the system of any of claims 1 to 10, the tube being receivable in a tube channel of a MIS instrument and comprising:
a first flow channel;
a second flow channel, fluidically separated from the first flow channel; and
a connector, at a proximal end of the tube, connectable to at least two syringe outlets,
a first of the at least two syringe outlets being connectable to the first flow channel, and a second of the at least two syringe outlets being connectable to the second flow channel, so that efflux from the at least two outlets is prevented from combining before reaching a distal end of the tube

13. A tube according to claim 12, wherein the tube comprises a limiter configured to engage with a proximal end of a tube channel of a MIS instrument; optionally wherein the tube is configured to project, distally, from a tube channel of a MIS instrument when the limiter engages a proximal end of a tube channel of the MIS instrument.

14. A tube according to claim 13, wherein the limiter comprises a flange having a diameter greater than a diameter of the tube channel.

15. A kit of parts, comprising a tube according to any of claims 12, 13 and 14, and a syringe comprising:
a first chamber having a first fluid outlet coupled to the first flow channel, the first chamber containing a first constituent; and
a second chamber having a second fluid outlet coupled to the second flow channel, the second chamber containing a second constituent,
wherein the first constituent and second constituent, when combined, form a haemostatic agent.
